# EUROPEAN PATENT SPECIFICATION

(11) **EP 1 181 935 B1**
(45) Date of publication and mention of the grant of the patent: **21.09.2005**
(21) Application number: 01112981.4
(22) Date of filing: 11.01.1996
(51) Int. Cl.: A61K 31/5513, A61P 25/18, A61P 25/22, A61P 25/24

(54) **Use of Olanzapine for the treatment of mental disorders resulting from cerebrovascular disorders**
Verwendung von Olanzapin zur Behandlung von mentalen Erkrankungen verursacht durch zerebrovaskuläre Erkrankungen
Utilisation d'Olanzapine pour le traitement de maladies mentales résultant de maladies cérébrovasculaires

(30) Priority: 12.01.1995 JP 319195
(43) Date of publication of application: 27.02.2002
(62) Divisional of application: 96100360.5
(73) Proprietor: SUMITOMO PHARMACEUTICALS COMPANY, LIMITED, Osaka-fu (JP)
(72) Inventor: Otomo, Eiichi, Tama-shi, Tokyo-to (JP)
(74) Representative: Viering, Jentschura & Partner

(56) References cited:
- EP-A- 0 437 790
- EP-A- 0 454 436
- WO-A-94/18196
- WO-A-96/38151
- DATABASE BIOSIS [Online] BIOSCIENCES INFORMATION SERVICE, PHILADELPHIA, PA, US; 1993 ANDO KIYOSHI ET AL: "Dependence study on LY170053 in rhesus monkeys and rats." Database accession no. PREV199396128181 XP002185175 & PRECLINICAL REPORTS OF THE CENTRAL INSTITUTE FOR EXPERIMENTAL ANIMALS, vol. 19, no. 2, 1993, pages 73-92, ISSN: 0385-8502
- MOORE N A ET AL: "THE BEHAVIORAL PHARMACOLOGY OF OLANZAPINE, A NOVEL ATYPICAL ANTIPHSYCHOTIC AGENT" JOURNAL OF PHARMACOLOGY AND EXPERIMENTAL THERAPEUTICS, AMERICAN SOCIETY FOR PHARMACOLOGY AND, US, vol. 262, no. 2, 1 August 1992 (1992-08-01), pages 545-551, XP000573947 ISSN: 0022-3565
- OBERHOLZER A F ET AL: "SAFETY AND EFFECTIVENESS OF LOW-DOSE CLOZAPINE IN PSYCHOGERIATRIC PATIENTS: A PRELIMINARY STUDY" INTERNATIONAL PSYCHOGERIATRICS, SPRINGER PUBLISHING INTERNATIONAL, US, vol. 4, no. 2, 1992, pages 187-195, XP000570690 ISSN: 1041-6102
- BARNES R.: 'Efficacy of antipsychotic medications in behaviorally disturbed dementia patients' AM.J.PSYCHIATRY vol. 139, no. 9, 1982, pages 1170 - 1174
- BERKOW R.: 'The Merck Manual of Diagnosis and Therapy, 15th Edition', 1987, MERCK SHARP AND DOME RESEARCH LABORATORIES, RAHWAY, N.J.

## Description

The present invention relates to a new therapeutic agent for treating mental disorders resulting from cerebrovascular disorder.

In cerebrovascular diseases such as cerebral infarction (cerebral thrombosis and embolism) and cerebral hemorrhage, a local ischemic condition occurs in the brain, which results in a cerebral organic or functional disorder such as defluxion of the nerve cells, ventricular expansion or the like. Therefore, in patients who have cerebrovascular disease, loss of intellectual function such as depressions of mneme and memory, or various mental disorders such as emotional disturbance, loss of spontaneity, and problem behavior will develop.

Although the pathophysiological mechanism leading to the mental disorders resulting from the cerebrovascular diseases is not known, it is considered that the disorder may occur due to the development of an abnormality in intracerebral serotonin and dopamine neural functions. Thus, the following findings have been reported: ischemia causes enhanced release of serotonin or dopamine, or decreases the content thereof in the brain (Chang et al.,J. Neurochem., 60, 1483, 1993; Luthman et al., Pharmacol. Biochem. Behav., 41, 231, 1991); it increases or decreases proteins (receptors) which receive these neurotransmitters and control neurotransmission (Robinson and Starkstein, J., Geriatr. Psychiatry, 22, 1, 1989; Bracha et al., Biol. Psychiatry, 25, 265, 1989; Nagasawa et al., J. Neorosci. Res., 33, 485, 1992). As both central serotonin and dopamine nerve systems are generally known to play an important role in developing emotion and mental function, dysfunction of these systems appears to be involved in developing the mental disorders resulting from cerebrovascular disease.

Application of a dopamine-2 blocker (a typical antipsychotic agent) has been investigated as a chemotherapeutic treatment of the mental disorders resulting from cerebrovascular disease, which demonstrated that the agent was effective to some degree in the treatment of problem behavior but, in some cases, resulted in a deterioration of emotional disturbances, loss of spontaneity, and the like. Accordingly, the agent is not a satisfactory therapeutic agent for the treatment of such conditions.

### SUMMARY OF THE INVENTION

Extensive studies directed towards the treatment of various mental disorders have led to the unexpected finding that Olanzapine which can block both serotonin-2 and dopamine-2 receptors exhibits very remarkable effects on the treatment of the mental disorders resulting from cerebrovascular disorder. The present invention is based on the above finding.

Thus, the present invention provides the use of Olanzapine for the preparation of a medicament for treating mental disorders resulting from cerebrovascular disorders.

### DETAILED EXPLANATION OF THE INVENTION

Mental disorders that can be expected to be improved or healed with the therapeutic agent of the invention include problem behavior such as aggressive behavior, mental excitement, fugue, delirium, hallucination and delusion, which are observed in patients with cerebral infarction such as upon cerebral thrombosis and embolism and cerebral hemorrhage; emotional disturbance such as anxiety, emotional tension, depressive mood, emotional incontinence, irritability, poor expression and bad humor; and loss of spontaneity such as reduced positiveness, reduced expression of desires, reduced initiative in activities, reduced interest in housework, amusement and pastime.

According to the present invention, the compound which can block both serotonin-2 and dopamine-2 receptors is: Olanzapine (Drug Data Report 1992, 14 (11), EP-A-454436) and therefore, can be used in accordance with the present invention.

The foregoing compound which can block both serotonin and dopamine receptors may be formulated to pharmaceutical formulations according to any of the methods known to those skilled in the art. For example, the compound described above may be combined with ordinary excipients, diluents or carriers to form tablets, capsules, suspensions or powders. Examples of the excipients, diluents or carriers suitable for such formulations include the following: filling and extending agents such as starch, sugar, mannitol, and silicon derivatives; binding agents such as carboxymethyl cellulose and other cellulose derivatives, alginate, gelatin and polyvinylpyrrolidone; wetting agents such as glycerol; disintegrating agents such as sodium bicarbonate; dissolution dilating agents such as paraffine; adsorption accelerating agents such as quaternary ammonium compounds; detergents such as acetyl alcohol and glycerol monostearate; adhesive carriers such as kaoline and bentonite; and lubricating agents such as talc, calcium and magnesium stearates, and solid polyethyleneglycol.

The foregoing compound can also be formulated as elixirs or solutions convenient for oral administration, or solutions suitable for parenteral administration (e.g. intramuscular, subcutaneous or intravenous route). Moreover, the above compound may be formulated in a sustained release dosage form. Depending on circumstances, the compound can be designed as a formulation which releases an active ingredient during a certain period of time, preferably only in the particular site of the intestinal tract. For example, such formulation may be covered with a coating, an envelope or a protecting matrix using a high molecular substance or wax.

The therapeutical dose of the compound of the present invention for human use, which can block both serotonin-2 and dopamine-2 receptors, will vary depending on such factors as the severity of condition of the patient, administration route and the related factors, and its determination is within the skill of a practicing physician. Typically, the acceptable effective dose for an adult is 0.05 to 500 mg/day, more typically 0.2 to 50 mg/day. Patients in need of such treatment will receive such dose once to 3 times a day, or more if necessary, for a period of time long enough to inhibit the diseases or disorders. For example, SM-9018 the compound claimed in the parent application EP 96 10 0360.5 (reference), may be orally administered at a dose ranging from 0.5 to 10 mg/adult patient/day. More specifically, SM-9018, when orally administered, may be administered at a dosage of 1mg once to 3 times a day for about 8 weeks, which may be followed by an increase in dosage to 2 mg, if the 8-week treatment failed to improve the conditions of the patients.

The foregoing compound which can be used in the present invention is either already being used as medicaments or is undergoing clinical trials.

The compound of the present invention is usually administered, in the form of pharmaceutical formulation as stated above, to patients having mental disorders resulting from cerebrovascular disorders. Specifically, such patients include those who were suffering from cerebral infarction such as cerebral thrombosis and embolism, and cerebral hemorrhage, whose symptoms are now stable for a month or more after onset, and yet who are still suffering from either problem behavior such as aggressive behavior, mental excitement, fugue or delirium, emotional disturbance such as anxiety, emotional tension, depressive mood, emotional incontinence or irritability, reduced spontaneity, hallucination and delusion, sleeping disorders, or somatic complaints such as the head ache, the head feeling heavy, hypochondriacal complaints or anorexia. The preferable mode of administration is oral administration.

The above-listed compound used in the present invention can be prepared according to the methods described in the publication mentioned above. For example, SM-9018 (reference) is described as Compound No.8 in Japanese Patent Publication (KOKAI) No.12379/1987, on page 21, and it may be prepared according to working examples therein.

The present invention will be illustrated in more detail with reference to the following preparations, formulations and examinations. However, they are representative, only.

### Preparation 1 Synthesis of SM-9018 (reference)

A mixture of 2.37 g (8.22 mmol) of N-(4-bromobutyl)-1,2-cis-cyclohexandicarboxyimide, 1.5 g (6.84 mmol) of 1-(1,2-benzisothiazol-3-yl)piperazine, 1.13 g (8.21 mmol) of potassium carbonate, 0.113 g (0.68 mmol) of potassium iodide and 25 ml of dry dimethylformamide (DMF) was heated in a warm bath at 90-100°C for 7 hours with stirring. The mixture was filtered to remove the solids, and the filtrate was concentrated under vacuum. The resultant oily residue (4.1 g) was purified by silica gel chromatography to give 2.48 g (5.81 mmol, 84.9%) of N-[4-[4-(1,2-benzisothiazol-3-yl)-1-piperazinyl]butyl]-1,2-cis-cyclohexandicarboxyimide. It was converted to a hydrochloride salt according to a conventional process, recrystallized in isopropyl alcohol to give SM-9018 [N-[4-[4-(1,2-benzisothiazol-3-yl)-1-piperazinyl]butyl]-1,2-cis-cyclohexandicarboxyimide hydrochloride]. mp. 184-185°C.

### Formulation 1 Gelatin capsules

Using the following ingredients, a hard gelatin capsule is formulated.

| Ingredients | Amount (mg/capsule) |
|---|---|
| SM-9018 (reference) | 1 |
| Starch, NF | 0-400 |
| Lactose | 0-400 |
| Magnesium stearate | 0-10 |

The above ingredients are homogeneously mixed, passed through a sieve, and then filled in a hard gelatin capsule.

### Formulation 2 Tablets

| Ingredients | Amount (mg/capsule) |
|---|---|
| SM-9018 (reference) | 1 |
| Microcrystalline cellulose | 0-200 |
| Silicon dioxide | 0-200 |
| Stearate | 0-5 |

The above ingredients are homogeneously mixed and compressed to form tablets.

### Examination 1 Clinical trial of SM-9018 (reference) for mental disorders associated with cerebrovascular disorders.

SM-9018 was administered to patients with cerebral infarction or hemorrhage accompanied by various mental disorders described above to examine whether the mental disorders will be improved or not.

### [1] Mode of administration

One mg/dosage of SM-9018 was orally administered once (Step I), twice (Step II) or 3 times a day (Step III) for up to 2 weeks after the administration was started and, when the treatment was effective, administration thereof was continued. When no effect was detected but the administration did not harm the patients, the dose of the compound administered was increased to 1 mg x 2 times/ day (Step I), 2 mg x 2 times/day (Step II), and 2 mg x 3 times/day (Step III).

### [2] Administration periods: 8 weeks

### [3] Results of the trials

Improvement of the mental disorders was evaluated by categorizing into 4 grades: i.e. 1) "excellent", 2) "moderate", 3) "slight", or 4) "unchanged" or other. Among the results obtained, "the number of patients categorized to 1) + 2) divided by the number of patients tested" for each symptom is summarized in the following Tables 1 to 3.

### (i) Problem behaviors

**Table 1**

| | Step I | Stdp II | Step III |
|---|---|---|---|
| Aggressive behavior | 2/7 | 3/4 | 2/5 |
| Mental excitement | 2/14 | 4/10 | 3/8 |
| Fugue | 1/6 | 1/3 | 0/3 |
| Delirium | 1/4 | 2/5 | 1/4 |
| Total problem | | | |
| behavior | 2/18 | 5/10 | 3/10 |

### (ii) Emotional disturbances

**Table 2**

| | Step I | Step II | Step III |
|---|---|---|---|
| Anxiety | 3/22 | 4/23 | 6/19 |
| Emotional tension | 2/18 | 3/18 | 5/15 |
| Depressive mood | 4/25 | 8/23 | 6/18 |
| Emotional incontinence | 2/13 | 3/13 | 2/10 |
| Irritability | 4/25 | 5/19 | 5/16 |
| Bad humor | 4/18 | 9/21 | 6/16 |
| Total emotional disorders | 8/33 | 11/28 | 10/20 |

### (iii) Reduced spontaneity

**Table 3**

| | Step I | Step II | Step III |
|---|---|---|---|
| Reduced positiveness to an influence | 5/30 | 6/23 | 3/17 |
| Reduced expression of desires | 3/29 | 5/23 | 3/16 |
| Reduced initiative in activities | 3/30 | 5/26 | 6/17 |
| Reduced interest in housework, amusement, pastime | 4/31 | 6/27 | 8/16 |
| Total loss of spontaneity | 4/32 | 8/27 | 9/18 |

### (iv) Final global improvement rating

**Table 4**

| | No. of patients examined | Improvement rating | | |
|---|---|---|---|---|
| | | Excellent | Moderate | Slight |
| Step I | 35 | 2 | 5 | 20 |
| Step II | 28 | 3 | 10 | 8 |
| Step III | 20 | 6 | 7 | 3 |

According to the above global evaluation, it is evident that some improvement of those mental disorders ,including those which were judged as being "slight", was observed in about 77% of patients in Step I, about 75% in Step II, and about 80% in Step III.

## Claims

1. Use of Olanzapine for the manufacture of a medicament for treating mental disorders resulting from cerebrovascular disorder.

2. Use according to claim 1, wherein the amount of the compound to be administered is from 0.2 to 50 mg/adult patient/day.

3. Use according to claim 1 or 2, wherein the cerebrovascular disorder is cerebral infarction or hemorrhage.

4. Use according to any one of claims 1 to 3, wherein the mental disorder resulting from the cerebrovascular disorder is an emotional disturbance, reduced spontaneity, problem behavior, hallucination or delusion.

5. Use according to any one of claim 1 to 3, wherein the mental disorder resulting from the cerebrovascular disorder is an emotional disturbance or reduced spontaneity.

6. Use according to any one of claims 1 to 3, wherein the mental disorder resulting from the cerebrovascular disorder is reduced spontaneity.

## Patentansprüche

1. Verwendung von Olanzapin für die Herstellung eines Medikaments zur Behandlung von Geisteskrankheiten, die die Folge von cerebrovaskulärer Erkrankung sind.

2. Verwendung nach Anspruch 1, wobei die zu verabreichende Menge der Verbindung 0,2 bis 50 mg/erwachsenem Patienten/Tag beträgt.

3. Verwendung nach Anspruch 1 oder 2, wobei die cerebrovaskuläre Störung ein Hirninfarkt oder eine Blutung ist.

4. Verwendung nach einem der Ansprüche 1 bis 3, wobei die Geisteskrankheit, welche aus der cerebrovaskulären Erkrankung resultiert, eine seelische Erregung, verminderte Spontaneität, Verhaltensstörung, Halluzination oder Wahnvorstellung ist.

5. Verwendung nach einem der Ansprüche 1 bis 3, wobei die Geisteskrankheit, welche aus der cerebrovaskulären Erkrankung resultiert, seelische Erregung oder verminderte Spontaneität ist.

6. Verwendung nach einem der Ansprüche 1 bis 3, wobei die Geisteskrankheit, welche aus der cerebrovaskulären Erkrankung resultiert, eine verminderte Spontaneität ist.

## Revendications

1. Utilisation de l'olanzapine pour la fabrication d'un médicament pour le traitement de troubles mentaux résultant d'un trouble cérébrovasculaire.

2. Utilisation selon la revendication 1, où la quantité du composé à administrer est de 0,2 à 50 mg/patient adulte/jour.

3. Utilisation selon la revendication 1 ou 2, où le trouble cérébrovasculaire est un infarctus cérébral ou une hémorragie cérébrale.

4. Utilisation selon l'une quelconque des revendications 1 à 3, où le trouble mental résultant du trouble cérébrovasculaire est un trouble émotionnel, une réduction de la spontanéité, un problème de comportement, des hallucinations ou un délire.

5. Utilisation selon l'une quelconque des revendications 1 à 3, où le trouble mental résultant du trouble cérébrovasculaire est un trouble émotionnel ou une réduction de la spontanéité.

6. Utilisation selon l'une quelconque des revendications 1 à 3, où le trouble mental résultant du trouble cérébrovasculaire est une réduction de la spontanéité.
